(19) Europäisches Patentamt
European Patent Office
Office européen des brevets

(11) Publication number: 0 177 059 B1

(12) EUROPEAN PATENT SPECIFICATION

(45) Date of publication of patent specification: 16.01.91

(51) Int. Cl.[5]: C 07 C 275/62

(21) Application number: 85112618.5

(22) Date of filing: 04.10.85

# (54) Modified polyisocyanates with biuret structure and process for their preparation.

(30) Priority: 05.10.84 IT 2300384

(43) Date of publication of application:
09.04.86 Bulletin 86/15

(45) Publication of the grant of the patent:
16.01.91 Bulletin 91/03

(84) Designated Contracting States:
AT BE DE FR GB NL

(56) References cited:
US-A-3 954 714

(73) Proprietor: TECNOPART S.r.l.
31, Foro Buonaparte
Milan (IT)

(72) Inventor: Bianchin, Eugenio
7, via Monfenera
Treviso (IT)
Inventor: Longo, Giovanni
4, via Niccolò Tommaseo
I-30038 Spinea (Venezia) (IT)
Inventor: Lunardon, Gianflavio
26, via Milazzo
Padova (IT)

(74) Representative: Weinhold, Peter, Dr.
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

EP 0 177 059 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to modified polyisocyanates having biuret structure and to a process for their preparation.

As it is known, polyisocyanates having biuret structure are obtained by reacting three moles of an organic diisocyanate with one mole of an adduct-producing agent.

The term "organic diisocyanate" as used in the present specification and in the claims herein, is intended to mean the compounds having general formula:

$$OCN—R_1—NCO \qquad (II)$$

wherein $R_1$ is an aliphatic radical, a cycloaliphatic radical or a cycloaliphatic radical having aliphatic substituents, said radicals preferably containing up to 20 carbon atoms and being optionally substituted with one or more groups selected from halogen, such as fluorine, chlorine, bromine, iodine; $NO_2$, alkyl and alkoxy radicals.

Examples of organic diisocyanates are: ethylene-diisocyanate, butylene diisocyanate, pentamethylene diisocyanate, hexamethylene diisocyanate, cyclopentylene-1,3-diisocyanate, cyclohexylene-1,4-diisocyanate, cyclohexylene-1,2-diisocyanate, hexahydro xylylene-diisocyanate, dichloro-hexamethylenediisocyanate; dicyclohexyl-4,4'-diisocyanate, 1,2-di(isocyanatomethyl)cyclobutane, 1-methyl-2,4-diisocyanato-cyclohexane, 1-methyl-2,6-diisocyanato-cyclohexane, etc.; aliphatic diisocyanates containing ether groups such as 1,3-bis-(γ-isocyanatopropoxy)-2,2-dimethyl-propane, etc.

Suitable adduct-producing agents are: water, water contained in compounds in the form of crystallization water or in statu nascendi; formic acid, chlorohydrates, aqueous alcohols, monovalent tertiary alcohols, dicarboxylic acids which can be converted into their anhydrides such as oxalic acid, maleic acid; salicyclic acid etc., hydrogen sulphide, primary and secondary amines, diamines, polyamines, etc.

Processes for the production of polyisocyanates having biuret structure using the above mentioned adduct-producing compounds are described in US—A—3,124,605; 3,350,438; 3,358,010; 3,392,183; 3,862,973; 3,896,154; 4,051,165; 4,147,714; 4,176,132; 4,218,390; 4,320,068, etc.

As it is known, the reaction of an organic diisocyanate having formula (II) with an adduct-producing agent leads to the formation of an ureic bond ($R_1$—NH—CO—NH—$R_1$) which reacts further with isocyanate groups to form a tri- or poly-functional polyisocyanate.

When water is used as adduct-producing agent the reaction can be represented as follows:

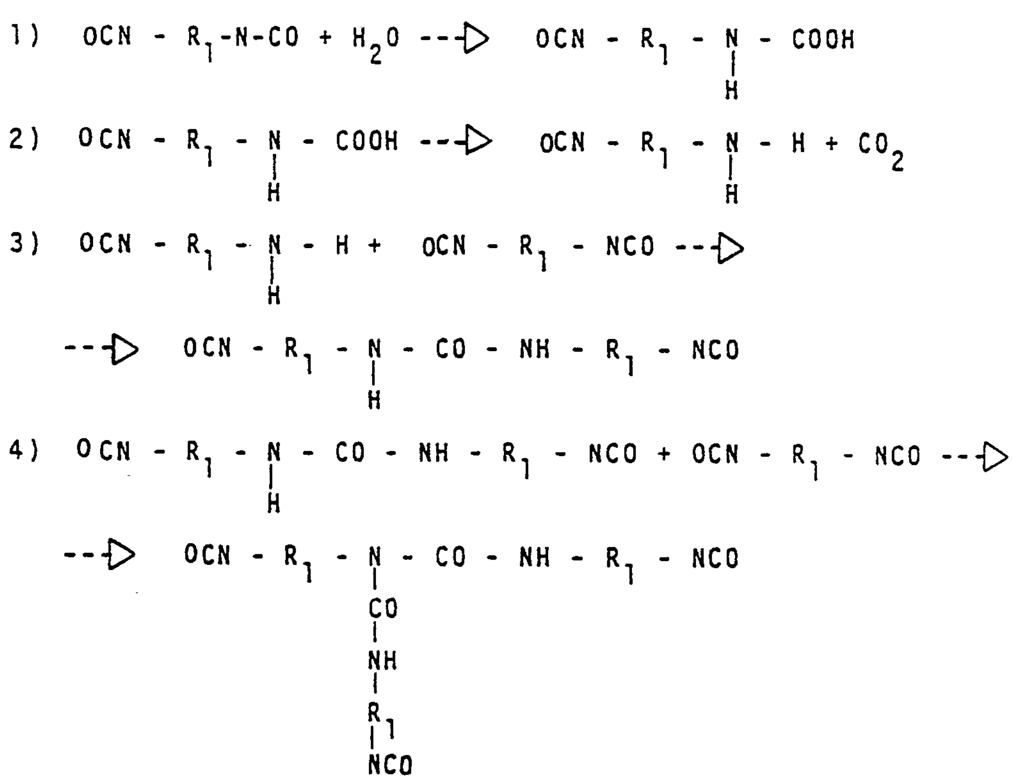

The thus formed polyisocyanate having biuret structure is soluble in common organic solvents such as toluene, xylene, acetic acid esters, etc.

The hereinabove reported reactions 1—4 are consecutive, but bifunctional urea or oligomers containing ureic bonds which form during the reactions can be solid or only slightly soluble in the reaction medium and/or with the thus formed polyisocyanate product. This makes the final product muddy and involves problems of deposition and clogging in the production plant.

However, while, on the one hand, it is desirable that the ureic groups (NH—CO—) react with the isocyanate groups (OCN—), a polyisocyanate devoid of free ureic groups on the other hand has a high viscosity, especially when its molecular weight is high.

A very viscous polymer is only scarcely miscible with other resins or soluble in solvents and therefore it is not suitable for being used, for example, in paints without adversely affecting the film-forming and physical properties of the corresponding paint.

It has been proposed to use an excess of the organic diisocyanate with respect to the adduct-producing agent to produce polyisocyanates having biuret structure, devoid of free ureic groups and with low molecular weight.

Thus, US—A—3,903,127 describes a process for the preparation of polyisocyanates with biuret structure wherein the molar ratio diisocyanate/adduct producing agent is at least 11:1. This solution, however, is not free of drawbacks, due both to the need of stripping off the free diisocyanate and to the eventual thermal polymerization of the excess diisocyanate with formation of coloured products such as uretidons, isocyanates, carbodiamide bonds, etc.

Another inconvenience in the preparation of polyisocyanates having biuret structure is the fact that the adduct-producing agents generally are only scarcely soluble or completely insoluble in the diisocyanate. Thus, for example, in case water is used as adduct-producing agent, due to the low miscibility of water in organic diisocyanates emulsions are obtained. The reaction between water and the diisocyanate monomer dissolved in the aqueous phase leads to the formation of polyureas as by-products which precipitate. The precipitation of polyureas is not only economically disadvantageous, but it also reduces the commercial value of the final polyisocyanate.

The amount of polyurea by-products varies according to temperature, stirring speed, ratio diisocyanate/water, etc., but generally it always exceeds 0.5% by weight with respect to the final polyisocyanate. The amount of polyurea can be minimized by adding 40 moles or more of diisocyanate monomer per mole of water. A large excess of diisocyanate, however, does not completely eliminate the formation of polyureas and, in addition, it involves the problem of having to remove the large excess of non-reacted monomer.

In order to prevent the formation of polyureas as by-product, US—A—4,028,392 proposes to prepare a polyisocyanate prepolymer by reacting a diisocyanate with water in an organic hydrophilic solvent capable of dissolving both the water and the organic diisocyanate to form a homogeneous phase. The hydrophilic solvents proposed in this patent must not be reactive with the isocyanate group and are selected from esters of carboxylic acids, esters-amides of phosphoric acid, ketones, nitriles ethers, etc., such as methyl-cellosolve® acetate, cellosolve® acetate, methyl-butyl-ketone, trimethyl-phosphate, dimethyl-formamide, propionitrile, adiponitrile, etc.

This technique too, however, is not free of drawbacks, due to the need to remove said solvents at the end of the reaction. In fact, incomplete removal of the solvent affects the physical properties of the products obtained from polyisocyanates, such as varnishes or paints.

An object of the present invention is to prepare a polyisocyanate having biuret structure which does not have the drawbacks just described.

Another object of the present invention is to provide a process for the preparation of a polyisocyanate having biuret structure which has a low molecular weight, a narrow molecular weight distribution and a low viscosity so as to be miscible with other resins and solvents, and which is free of polyureas.

Still another object of the present invention is to provide a process for the preparation of a polyisocyanate having biuret structure wherein precipitation of polyurea does not occur.

A further object of the present invention is to provide a process for the preparation of a polyisocyanate having high quality biuret structure and being essentially colorless.

According to the present invention, these and other objects are achieved by a modified polyisocyanate having biuret structure containing terminal urethane groups and having general formula.

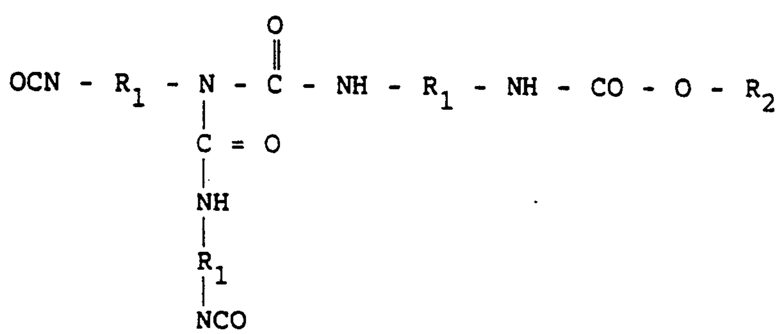

(I)

wherein $R_1$ is an aliphatic radical, a cycloaliphatic radical or a cycloaliphatic radical having aliphatic

substituents, said radicals preferably containing up to 20 carbon atoms and being optionally substituted with one or more groups selected from halogen, such as fluorine, chlorine, bromine, iodine; $NO_2$, alkyl and alkoxy radicals; and $R_2$ can have the same meanings as $R_1$ or is the radical:

$$R_3(OC_nH_{2n})_x\text{—} \qquad (IV)$$

wherein $R_3$ is an alkyl or acyl radical having up to 18 carbon atoms, n is an integer ranging from 2 to 4 and x is an integer ranging from 1 to 50.

According to another embodiment of the present invention, there is provided a process for the preparation of modified polyisocyanates with biuret structure having formula (I), which consists in reacting water or a compound which splits off water with an organic diisocyanate, in a molar ratio diisocyanate/water lower than 10:1, preferably between 5:1 and 10:1, in the presence of a compatibilizing agent in an amount of less than 10 parts by weight per one part by weight of water, said compatibilizing agent having a boiling point exceeding 80°C and a hydrogen atom reactive towards the isocyanate group, as it can be determined by means of Zerewitinoff's method, and being of the formula

$$R_2\text{—}OH \qquad (III)$$

wherein the OH group is a primary hydroxyl group of high polarity and $R_2$ is as defined above.

The process according to the present invention does not only prevent the formation of polyureas as by-products, but it also allows to obtain modified polyisocyanates with biuret structure having a narrower molecular weight distribution, a viscosity of less than 10,000 mPa.s at 25°C and a low molecular weight, particularly suitable for being used in the paint field.

The compatibilizing agents used in the present invention are the ones which are capable of forming a homogeneous phase with water and diisocyanate under the reaction conditions and for the whole reaction period.

The compatibilizing agents having boiling points ranging from 80 to 200°C are preferred, so that, under the reaction conditions, they do not evaporate before interacting with the, isocyanate groups.

The quantity of said compatibilizing agents is less than 10 parts by weight of compatibilizing agent per one part by weight of water and can vary according to the type of diisocyanate, to the used solvents and to the reaction conditions.

Weight ratios water/compatibilizing agents ranging from 9.1 to 1.9 particularly from 2.1 to 1.2 are preferred. In practice a weight ratio water/compatibilizing agent of 1.1 is particularly preferred.

The use of said compatibilizing agents having a hydrogen atom reactive with the isocyanate group makes it possible to obtain polyisocyanates having the required properties, although molar ratios of diisocyanate and water lower than those known in the art may be used. As a consequence, the quantity of residual diisocyanate to be evaporated at the end of the reaction is by far smaller. Furthermore, since the compatibilizing agents are reactive with isocyanate groups, they are immobilized under formation of urethane bonds and therefore their removal at the end of the reaction is not necessary.

Examples of compatibilizing agents which can advantageously be used in the process of the present invention are: polyoxyethylene monoethyl-ether, polyoxyethylene monobutylether, polyoxybutylene monoethylether, polyoxybutylene monobutylether, polyoxyethylene monoacetate, polyoxybutylene monoacetate, cellosolve®, butyl-cellosolve® and higher homologues, ethylene glycol acetate, butylene glycol acetate, monoethylester of glycolic acid, monobutylester of glycolic acid, etc.

According to the present invention the water used as adduct producing agent can be employed either as such or in the form of compounds which split off water, such as compounds containing crystallization water, for instance sodium sulphate, oxalic acid and formaldehyde hydrate, dicarboxylic acids which can easily be converted into anhydrides, such as, for example, maleic acid, and salicylic acid, etc.

Organic diisocyanates which can be used in the process according to the present invention are the ones having general formula (II) as reported hereinabove. Hexamethylene diisocyanate is particularly preferred.

The reaction for the formation of biuret can be carried out both in the presence and in the absence of catalysts. In the former case the catalysts generally used in the reaction between water and diisocyanates can be used.

Examples of said catalysts are: tertiary amines, such as triethylene diamine, N-ethyl-ethylene-imine, tetramethylguanidine, dimethylcyclohexylamine, dimethylethanolamine, etc; and organometallic activators such as dibutyltin dilaurate, tin octoate, cobalt naphthenate, vanadium acetylacetonate, dimethyltin diethylhexanoate and mixtures thereof.

The preferred catalysts are the organometallic ones, since they are much more active in the process of the present invention. However, they have to be used in very low concentrations, generally less than 0.5% by weight with respect to diisocyanate, in order to avoid that the product passes from low and applicatively acceptable viscosity, obtained at the end of the reaction, to viscosities increasing with time, especially if the product is stored at temperatures exceeding room temperature or under continuous stirring. For this reason it is generally preferable not to use catalyst during the stage of biuret preparation. Catalysts of high activity preferred in the process according to the present invention are tin octoate and dibutyltin dilaurate.

During the reaction, it is preferred to maintain the reaction medium under vigorous stirring in order to facilitate the dispersion of the adduct-producing agent in the diisocyanate and to disfavour the separation of ureas and polyureas.

The reaction temperature generally ranges from 80°C to 200°C, and preferably from 130°C to 190°C, in order to obtain a high reaction rate even in the absence of catalysts, and at the same time, to avoid the yellowing of the product.

It is preferred to first carry out the reaction of water and compatibilizing agent and to then add the mixture thus obtained to the diisocyanate, deaerated and preheated at the above reported reaction temperature, under continuous stirring. The mixture of water and compatibilizing agent is added in a way such that the molar ratio diisocyanate/water in total ranges between 5:1 and 10:1, preferably around 7:1. Furthermore, the feed rate is adjusted so as to maintain, as much as possible, a constant reaction temperature.

When the addition of the water-compatibilizing agent mixture is over, the reaction medium is kept under stirring at constant temperature for the minimum time necessary for the completion of the reaction, which can be determined, for example, by testing the viscosity.

At the end of the reaction, the excess of diisocyanate is recovered from the reaction mixture at the lowest possible temperature in order to avoid formation of oligomers and/or coloured products. A thin layer evaporator or a molecular distillator can, for example, be used. Thus a modified polyisocyanate with biuret structure containing terminal urethane groups and having a free diisocyanate content of less than 0.5% by weight is obtained.

As mentioned above, the process according to the present invention is carried out in homogeneous phase in the presence of a compatibilizing agent for the diisocyanate and the adduct-producing agent, which compatibilizing agent is reactive towards diisocyanate. The final product mainly consists of diisocyanate biuret and, possibly, its higher homologues, modified by the presence of terminal urethane groups and derived from the employed compatibilizing agent.

The obtained product generally has a viscosity at 25°C of less than 10,000 mPa.s, preferably lower than 5,000 mPa.s, a narrow molecular weight distribution, a titre of free NCO ranging from 21 to 23% and a complete solubility in toluene at 10% by weight for at least 4 hours.

To the reaction system there can be added discoloration inhibitors, UV stabilizers, catalysts and other additives well known to those skilled in the art for the production of polyisocyanates.

The following examples further illustrate the present invention without being a limitation of embodiments thereof.

Unless otherwise stated, all parts, percentages and ratios referred to in the examples are by weight.

## Example 1

0.17 parts of catalyst (dibutyltin dilaurate) are added to 350 parts of hexamethylene diisocyanate contained in a flask provided with stirrer, reflux condenser, thermometer and feeding inlets and the mixture is heated under a light flow of dry $N_2$ up to 140°C. 10.7 parts of a previously prepared 1:1 mixture of water/butylcellosolve® are then added dropwise and under vigorous stirring.

The speed at which the mixture is fed is such as to maintain the temperature within ±°C.

Precipitates or cloudiness are not observed during the reaction, which manifests itself by a continuous, regular evolution of $CO_2$ gas. After the addition is complete the reaction mixture is kept at the indicated temperature and under stirring for a further 15 minutes. The excess monomer is then recovered by distillation at 140°C and 0.067—0.133 kPa (0.5—1 mmHg).

The final product is a liquid (coloration lower than 1 Gardner) having a free NCO titre of 22.8%, a viscosity at 25°C of 1830 mpa.s and a residual hexamethylene diisocyanate content lower than 0.5% by weight, determined by chromatographic analysis.

When the resulting product is dissolved in 10% toluol, it does not show any turbidity even after 4 hours.

## Example 2

Using the same procedure and quantities as in example 1 except for the absence of catalyst and heating the reaction mixture up to 180°C, a clear liquid final product (colour less than 1 Gardner) having a titre of free NCO of 22.6%, a viscosity at 25°C of 1580 mPa.s and a residual hexamethylene diisocyanate content lower than 0.5%, determined by means of chromatographic analysis, is obtained.

When the resulting product is dissolved in 10% toluene, it does not show any turbidity even after 4 hours.

## Example 3

Using the same procedure and quantities as in example 1, expect for the use of 12.5 parts of a 1:1 mixture of water/butylcellosolve®, a clear liquid final product (colour less than 1 Gardner) having a titre of free NCO of 21:6%, a viscosity at 25°C of 4,400 mPa.s and a residual hexamethylend diisocyanate content lower than 0.5%, determined by means of chromatographic analysis, is obtained.

The product solution in 10% toluene does not show any turbidity after 4 hours.

Example 4

By using the same procedure and quantities as in example 3 but employing a 1:1 water/ethylene glycol acetate mixture, a clear liquid final product (colour less than 1 Gardner), having a free NCO titre of 21.9%, a viscosity at 25°C of 3800 mPa.s and a residual hexamethylene diisocyanate content lower, than 0.5%, determined by means of chromatographic analysis, is obtained.

The product solution in 10% toluene does not show any turbidity after 4 hours.

Example 5

By using the same procedure and quantities as in example 4 but employing a water/butylester of glycolic acid mixture, a clear final product (colour less than 1 Gardner) having, a free NCO titre of 22.5%, a viscosity at 25°C of 4500 mPa.s and a residual hexamethylene diisocyanate content lower than 0.5%, determined by means of chromatographic analysis, is obtained.

The product solution in 10% toluene does not show any turbidity after 4 hours.

**Claims**

1. Modified polyisocyanate with biuret structure containing terminal urethane groups and having general formula:

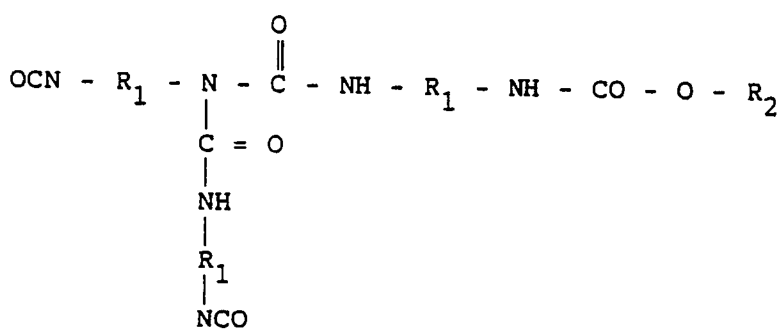

(I)

wherein $R_1$ is an aliphatic radical, a cycloaliphatic radical or a cycloaliphatic radical having aliphatic substituent(s), said radicals optionally being substituted with one or more groups selected from halogen, such as fluorine, chlorine, bromine, iodine; $NO_2$, alkyl and alkoxy radicals; and $R_2$ can have the same meanings as $R_1$ or is the radical:

$$R_3(OC_nH_{2n})_x\text{—} \qquad \text{(IV)}$$

wherein $R_3$ is an alkyl or acyl radical having up to 18 carbon atoms, n is an integer ranging from 2 to 4 and x is an integer ranging from 1 to 50.

2. Modified polyisocyanate according to claim 1, having a viscosity at 25°C of less than 10,000 mPa.s, and preferably less than 5,000 mPa.s, and a titre of free NCO groups of from 21 to 23%.

3. Process for the preparation of modified polyisocyanates according to claim 1, which consists in reacting water or a compound which splits off water with an organic diisocyanate, in a molar ratio diisocyanate/water lower than 10:1, in the presence of a compatibilizing agent in an amount of less than 10 parts by weight per one part by weight of water, said compatibilizing agent having a boiling point exceeding 80°C and a hydrogen atom reactive towards the isocyanate group, as it can be determined by means of Zerewitinoff's method, and being of the formula

$$R_2\text{—}OH \qquad \text{(III)}$$

wherein the OH group is a primary hydroxyl group of high polarity and $R_2$ is as defined in claim 1.

4. Process according to claim 3, wherein the compatibilizing agent has a boiling point ranging from 80° to 200°C.

5. Process according to claim 3, wherein the weight ratio water/compatibilizing agent ranges from 9.1 to 1.9 preferably from 2:1 to 1:2, and particularly is 1:1.

6. Process according to any of claims 3 to 5, wherein the molar ratio diisocyanate/water ranges from 5:1 to 10:1 and particularly is about 7:1.

7. Process according to claim 3, wherein the compatibilizing agent is butyl cellosolve®.

8. Process according to any of claims 3 to 7, wherein the reaction is carried out at temperatures ranging between 80° and 200°C, optionally in the presence of an organometallic catalyst such as tin octoate or dibutyltin dilaurate.

**Patentansprüche**

1. Modifiziertes Polyisocyanat mit Biuretstruktur, das Urethanendgruppen enthält und die allgemeine Formel aufweist:

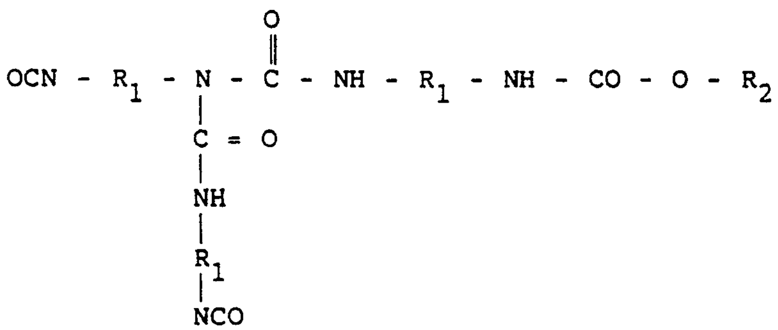

(I)

worin $R_1$ eine aliphatische Gruppe, eine cycloaliphatische Gruppe oder eine cycloaliphatische Gruppe mit einem oder mehreren aliphatischen Substituenten ist, wobei diese Gruppe wahlweise mit einer oder mehreren Gruppen, ausgewählt aus Halogen, wie Fluor, Chlor, Brom, Jod; $NO_2$, Alkyl- und Alkoxygruppen substituiert sein kann, und $R_2$ die gleichen Bedeutungen wie $R_1$ haben kann oder der Rest

$$R_3(OC_nH_{2n})_x\text{—} \qquad \text{(IV)}$$

ist, worin $R_3$ ein Alkyl- oder Acylrest mit bis zu 18 Kohlenstoffatomen ist, n eine ganze Zahl im Bereich von 2 bis 4 ist und x eine ganze Zahl im Bereich von 1 bis 50 ist.

2. Modifiziertes Polyisocyanat nach Anspruch 1 mit einer Viskosität bei 25°C von weniger als 10 000 mPa.s, vorzugsweise weniger als 5 000 mPa.s, und einem Titer freier NCO-Gruppen von 21 bis 23 %.

3. Verfahren zur Herstellung modifizierter Polyisocyanate nach Anspruch 1, das besteht:im Umsetzen von Wasser oder einer Wasser abspaltenden Verbindung mit einem organischen Diisocyanat in einem molaren Verhältnis von Diisocyanat: Wasser unter 10:1 in Anwesenheit eines verträglich machenden Mittels in einer Menge unter 10 Gew.-Teilen pro Gew.-Teil Wasser, wobei das verträglich machende Mittel einen Siedepunkt über 80°C hat und ein gegenüber Isocyanatgruppen reaktionsfähiges Wasserstoffatom aufweist, wie dies durch die Zerewitinoff-Methode bestimmt werden kann, und die Formel

$$R_2\text{—}OH \qquad \text{(III)}$$

hat, worin die OH-Gruppe eine primäre Hydroxylgruppe hoher Polarität ist und $R_2$ wie in Anspruch 1 definiert ist.

4. Verfahren nach Anspruch 3, worin das verträglich machende Mittel einen Siedepunkt im Bereich von 80 bis 200°C hat.

5. Verfahren nach Anspruch 3, worin das Gewichtsverhältnis von Wasser:verträglich machenden Mittel im Bereich von 9:1 bis 1:9, vorzugsweise von 2:1 bis 1:2 und insbesondere bei 1:1 liegt.

6. Verfahren nach irgendeinem der Ansprüche 3 bis 5, worin das molare Verhältnis von Diisocyanat:Wasser im Bereich von 5:1 bis 10:1 und insbesondere bei etwa 7:1 liegt.

7. Verfahren nach Anspruch 3, worin das verträglich machende Mittel Butyl-Cellosolve® ist.

8. Verfahren nach irgendeinem der Ansprüche 3 bis 7, worin die Reaktion bei Temperaturen im Bereich zwischen 80 und 200°C, wahlweise in Anwesenheit eines Organometall-Katalysators, wie Zinnoctoat oder Dibutylzinndilaurat, durchgeführt wird.

**Revendications**

1. Polyisocyanate modifié ayant la structure du biuret contenant des groupements terminaux uréthane et ayant la formule générale suivante:

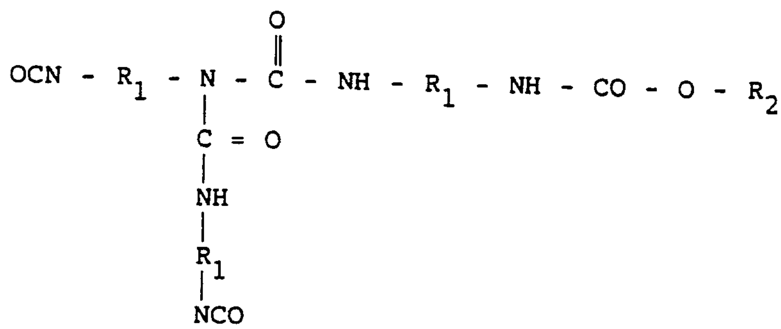

(I)

dans laquelle $R_1$ est un radical aliphatique, un radical cycloaliphatique ou un radical cycloaliphatique ayant des substituants aliphatiques, lesquels radicaux pouvant être éventuellement substitués avec un ou plusieurs groupements choisis parmi les halogènes, tels que le fluor, le chlore, le brome, l'iode; $NO_2$, les radicaux alkyle et alcoxy; et $R_2$ ayant la même signification que $R_1$ ou étant le radical:

$$R_3(OC_nH_{2n})_x\text{—} \qquad (IV)$$

dans laquelle $R_3$ est un radical alkyle ou acyle ayant jusqu'à 18 atomes de carbone, n est un nombre entier compris entre 2 et 4 et x est un nombre entier compris entre 1 et 50.

2. Polyisocyanate modifié selon la revendication 1, ayant une viscosité à 25°C inférieure à 10 000 mPa.s, et de préférence inférieure à 5 000 mPa.s, et un taux de groupements NCO libres compris entre 21 et 23%.

3. Procédé pour la préparation de polyisocyanates modifiés selon la revendication 1, qui consiste à faire réagir de l'eau, ou un composé qui libère de l'eau, avec un diisocyanate organique, dans un rapport molaire diisocyanate/eau inférieur à 10/1, en présence d'un agent de compatibilité dans une quantité inférieure à 10 parties en poids pour une partie en poids d'eau, lequel agent de compatibilité ayant un point d'ébullition supérieur à 80°C et un atome d'hydrogène réactif envers le groupement isocyanate, comme cela peut être déterminé au moyen de la méthode de Zerewitinoff, et étant de la formule

$$R_2\text{—}OH \qquad (III)$$

dans laquelle le groupement OH est un groupement hydroxyle primaire de forte polarité et $R_2$ tel que défini dans la revendication 1.

4. Procédé selon la revendication 3, dans lequel l'agent de compatibilité a un point d'ébullition compris entre 80°C et 200°C.

5. Procédé selon la revendication 3, dans lequel le rapport en poids entre l'eau et l'agent de compatibilité est compris entre 9:1 et 1:9, de préférence entre 2:1 et 1:2, et spécialement de 1:1.

6. Procédé selon l'une quelconque des revendications 3 à 5, dans lequel le rapport molaire entre le diisocyanate et l'eau est compris entre 5:1 et 10:1 et plus particulièrement lorsqu'il est aux environs de 7:1.

7. Procédé selon la revendication 3, dans lequel l'agent de compatibilité est le butyl-Cellosolve®.

8. Procédé selon l'une quelconque des revendications 3 à 7, dans lequel la réaction est effectué à une température comprise entre 80°C et 200°C, éventuellement en présence d'un catalyseurs organométallique tel que l'octoate d'étain ou le dilaurate de dibutylétain.